# EUROPEAN PATENT SPECIFICATION

(11) **EP 4 050 619 B1**
(45) Date of publication and mention of the grant of the patent: **17.06.2026**
(21) Application number: 21382166.3
(22) Date of filing: 26.02.2021
(51) Int. Cl.: G16H 40/20, G16H 40/40

(54) **TROUBLESHOOTING BY PROXIMITY INTERACTION AND VOICE COMMAND**
FEHLERBEHEBUNG DURCH INTERAKTION IN DER NÄHE UND SPRACHBEFEHL
DÉPANNAGE PAR INTERACTION DE PROXIMITÉ ET COMMANDE VOCALE

(43) Date of publication of application: 31.08.2022
(73) Proprietor: F. Hoffmann-La Roche AG, 4070 Basel (CH); Roche Diagnostics GmbH, 68305 Mannheim (DE)
(72) Inventor: HURNI, Emanuel, 6343 Rotkreuz (CH); LI, Nan, 6343 Rotkreuz (CH); FERNANDEZ, Josep, 08174 Sant Cugat del Vallés (ES); MORALES ROJO DEL RIO, Jacobo, 08174 Sant Cugat del Vallés (ES)
(74) Representative: Mewburn Ellis LLP

(56) References cited:
- US-A1- 2016 246 364
- US-A1- 2020 411 176

## Description

### Technical Field

The present disclosure generally relates to the communication and notification needs in a laboratory setting.

### Background

Typical known laboratory systems endeavor to facilitate laboratory information monitoring practices in a laboratory setting. In these known laboratory systems, typically, information about the laboratory displayed to a user is tailored based on user movement as well as user proximity with respect to an information source such as, for example, a laboratory analyzer. However, the focus of these known laboratory systems has generally been placed on facilitating the viewing of information based solely on user proximity to the location of the information content.

For example, the laboratory system disclosed in US Pub 2016/246364 uses distance-based screen transition and interactions. Additionally, the laboratory system disclosed in US Pub 2017/0228508 focuses on retrieving medical data based on proximity between a medical monitoring device and a clinician's mobile device.

US 2020/411176 discloses a predictive maintenance method by applying analytic methods (expert driven and/or by machine learning) to data generated by the laboratory instrument(s)/system(s)

Therefore, there is a need to provide supporting laboratory information consultation activities in a laboratory setting in order to take into account the potential laboratory information needs of a laboratory user as well as the interaction and flow of information within the laboratory system.

### Summary

The use of the 'a' or 'an' can be employed to describe elements and components of the embodiments herein. This is done merely for convenience and to give a general sense of the inventive concepts. This description should be read to include one or at least one and the singular includes the plural unless it is obvious that it is meant otherwise.

The term 'laboratory instrument' or "laboratory device" as used herein can encompass any apparatus or apparatus component operable to execute and/or cause the execution of one or more processing steps /workflow steps on one or more biological samples and/or one or more reagents. The expression 'processing steps' thereby can refer to physically executed processing steps such as centrifugation, aliquotation, sample analysis and the like. The term 'instrument' can cover pre-analytical instruments, post-analytical instruments, analytical instruments and laboratory middleware.

The term 'post-analytical instrument' as used in the present description can encompass any apparatus or apparatus component that can be configured to perform one or more post-analytical processing steps/workflow steps comprising - but not limited to - sample unloading, transport, recapping, decapping, temporary storage/buffering, archiving (refrigerated or not), retrieval and/or disposal.

The term 'pre-analytical instrument' as used in the present description can encompass any apparatus or apparatus component that can be configured to perform one or more pre-analytical processing steps/workflow steps comprising - but not limited to - centrifugation, resuspension (e.g., by mixing or vortexing), capping, decapping, recapping, sealing, desealing, sorting, tube type identification, rack loading, sample loading, sample quality determination and/or aliquotation steps. The processing steps may also comprise adding chemicals or buffers to a sample, concentrating a sample, incubating a sample, and the like.

The term 'analyzer' / 'analytical instrument' as used in the present description can encompass any apparatus, or apparatus component, configured to obtain a measurement value. An analyzer can be operable to determine via various chemical, biological, physical, optical or other technical procedures a parameter value of the sample or a component thereof. An analyzer may be operable to measure the parameter of the sample or of at least one analyte and return the obtained measurement value. The list of possible analysis results returned by the analyzer comprises, without limitation, can be concentrations of the analyte in the sample, a digital (yes or no) result indicating the existence of the analyte in the sample (corresponding to a concentration above the detection level), optical parameters, DNA or RNA sequences, data obtained from mass spectrometry of proteins or metabolites and physical or chemical parameters of various types. An analytical instrument may comprise units assisting with the pipetting, dosing, and mixing of samples and/or reagents. The analyzer may comprise a reagent-holding unit for holding reagents to perform the assays. Reagents may be arranged for example in the form of containers or cassettes containing individual reagents or group of reagents, placed in appropriate receptacles or positions within a storage compartment or conveyor. It may comprise a consumable feeding unit. The analyzer may comprise a process and detection system whose workflow can be optimized for certain types of analysis. Examples of such analyzer can be clinical chemistry analyzers, coagulation chemistry analyzers, immunochemistry analyzers, urine analyzers, nucleic acid analyzers, used to detect the result of chemical or biological reactions or to monitor the progress of chemical or biological reactions.

The term 'laboratory middleware' as used in the present description can refer to any physical or virtual processing device configurable to control a laboratory instrument or system comprising one or more laboratory instruments in a way that workflow(s) and workflow step(s) can be conducted by the laboratory instrument/system. The laboratory middleware may, for example, instruct the laboratory instrument/system to conduct pre-analytical, post analytical and analytical workflow(s)/ workflow step(s). The laboratory middleware may receive information from a data management unit regarding which steps need to be performed with a certain sample. In some embodiments, the laboratory middleware can be integral with a data management unit, can be comprised by a server computer and/or be part of one laboratory instrument or even distributed across multiple instruments of the laboratory system. The laboratory middleware may, for instance, be embodied as a programmable logic controller running a computer-readable program provided with instructions to perform operations.

A system for presenting laboratory data to a laboratory user is presented. The system can comprise a perception component configured to continuously gather in-situ context data regarding a laboratory and laboratory user, a user modeling component configured to receive the in-situ context data from the perception component to create a user specific model for each laboratory user in the laboratory, a laboratory device awareness component configured to monitor the status, performance, alarms, and/or maintenance of the laboratory devices within the laboratory, a notification component configured to receive the in-situ context data from the perception component and the laboratory device status data from the laboratory device awareness component and to process and determine which of these data from the in-situ context data and the laboratory device status data are to be presented to the laboratory user, and a presentation component configured to present the processed data from the notification component, wherein the presented data comprises both public and private notifications of the data to the laboratory user.

The in-situ context data can be gathered by multi-channel sensors such as, for example, wearables worn by users in the laboratory, indoor positioning devices positioned throughout the laboratory, motion sensors positioned throughout the laboratory, laboratory user location data, laboratory user interactions, biometric characteristics of the laboratory user such as, for example, fingerprints, voice, iris, and facial, and combinations thereof.

The laboratory user has the ability to toggle between the private and public notification of data. The private and public data notifications can be predefined. For example, the private data notifications can be presented on smart phones, tablets, laptops, desktops, wearable smart devices, virtual space, or any combination thereof and under the highest security and privacy standards established in international regulations such as, for example, GDPR and HIPAA. The private data notifications can be also be an audible (e.g., a beep), visual, gustatory, olfactory, or tactile (e.g., a vibration) alert. Additionally, the public data notifications can be presented on monitoring displays positioned throughout the laboratory, voice assistant devices positioned throughout the laboratory, laboratory device displays, alarms (e.g., audible, visual, or tactile), or any combination thereof. The public data can be data that all laboratory users of the laboratory system may see.

The laboratory system can further comprise a database for storing the in-situ context data from the perception component and the laboratory device status data from the laboratory system awareness component. The data stored on the database can be used to generate statistics and/or projections in order to provide improvements in services between the clients and suppliers of the laboratory.

A method for presenting laboratory data to a laboratory user is also presented. The method can comprise loading a first initial user model, initializing multi-channel sensors throughout a laboratory to collect in-situ data as the data occurs, updating user models by using each individual laboratory user's work habits and usual laboratory activities, updating the in-situ data, determining if the laboratory user is interacting with a laboratory device with the laboratory, if the laboratory user is interacting with a laboratory device, providing device information to the laboratory user with private and public notifications based on the laboratory user's user model, determining if the laboratory user is in transit to perform a task in a laboratory, if the laboratory user is in transit, removing private notifications regarding the task to be performed to the laboratory user and providing the laboratory user with information on the task based on the updated in-situ data, terminating the method if requested by the user or otherwise repeat the above method steps until laboratory user requests termination, and saving the in-situ data and updating the user specific model.

The method can further comprise measuring and storing efficiency on a database as to how well the laboratory user completes the task.

The method can further comprise uploading the data currently being a private notification to the laboratory user to be a public notification to the laboratory user based on a laboratory user's request.

The method can further comprise downloading data currently a public notification to the laboratory user to a private notification of the laboratory user based on a laboratory user's request. In addition, the laboratory user has the ability to augment the information in the private space of the laboratory user.

The first initial user model can be based on the laboratory user's individual demographics, preferences, and laboratory role. This data can be supplied to the laboratory system upon start-up.

The in-situ data can comprise the laboratory user, the laboratory user's location, the laboratory user's activity, and the time.

The laboratory user's private notification can provide confidential information for the laboratory user.

The method can further comprise a) initializing laboratory systems after loading the first initial user models, b) updating a laboratory device awareness model, c) updating public user notifications with standard predefined configurations, d) updating private user notifications according to user preferences, e) determining if intervention by a laboratory user is needed, f) if no intervention is required by the laboratory user, repeating the updating steps c through l, g) if invention is required by the laboratory user, checking the current in-situ data to determine an appropriate laboratory user to provide invention and updating the private notification of that laboratory user to provide appropriate laboratory user information, h) determining if the laboratory user wishes to terminate the method, and i) if the laboratory user does not want to terminate the method, repeating steps 1 through r until the laboratory user wants to terminate the method.

### Brief Description of the Several Views of the Drawings

The following detailed description of specific embodiments of the present disclosure can be best understood when read in conjunction with the following drawings, where like structure is indicated with like reference numerals and in which:
Fig. 1 illustrates information interaction flow between entities in a laboratory according to an embodiment of the present disclosure.
Fig. 2 illustrates smart laboratory architecture according to an embodiment of the present disclosure.
Fig. 3 illustrates a flow diagram for presenting laboratory data to a laboratory user by troubleshooting using proximity interaction according to an embodiment of the present disclosure.
Fig. 4 illustrates a scenario of notifying laboratory users privately based on SLAT (Subject, Location, Activity, and Time) according to an embodiment of the present disclosure.
Fig. 5 illustrates a supporting novice laboratory user scenario according to an embodiment of the present disclosure.
Fig. 6 illustrates a supporting service representative scenario according to an embodiment of the present disclosure.
Fig. 7 illustrates a supporting laboratory manager scenario according to an embodiment of the present disclosure.

### Detailed Description

In the following detailed description of the embodiments, reference is made to the accompanying drawings that form a part hereof, and in which are shown by way of illustration, and not by way of limitation, specific embodiments in which the disclosure may be practiced. It is to be understood that other embodiments may be utilized and that logical, mechanical and electrical changes may be made.

Typical laboratory users constantly consult laboratory systems for various information for a variety of purposes throughout the day. However, different laboratory users may be interested in different types of laboratory information regarding laboratory performance, alarms, medical data, guidance, and the like depending on the context of use. For example, Table 1 depicts examples of the possible different types of laboratory information needed for the different laboratory user roles.

**Table 1. Example of information needs for different laboratory roles**

| **Role** | **Potential laboratory information needs** |
|---|---|
| Laboratory Manager | As a **laboratory manager,** I do not observe the operation of the laboratory at all times but want to get an up-to-date overview of my laboratory performance as well as the workload of my laboratory technicians. I also want to be notified about abnormal performance issues that concern me so that I can better coordinate and allocate my laboratory resources. |
| Laboratory Technician | As a **laboratory technician in a connected laboratory,** my workload is sometimes influenced by my co-worker's decision and vice versa. I want to work in a coordinated manner so that everyone is aware of others' decisions. |
| | As a **laboratory technician who shares workloads with others,** I want each of us to be notified of our tasks at the right time and right place so that we can coordinate better and work more efficiently. |
| | As an **inexperienced laboratory technician,** I want to receive more hints on the go and to learn as fast as possible |
| Service Representative | As a **service representative,** I visit customer laboratories to resolve issues and perform maintenance from time to time. I want to obtain smooth handover of issues from the laboratory and the right information to support my service. |

As it can be seen from Table 1, laboratory users need various information including information about other laboratory users in order to coordinate workload amongst the laboratory users as well as information about laboratory systems for monitoring laboratory performance. It should be noted that laboratory systems, including instruments and IT products, also have their own information needs. For example, in a laboratory with connected analytical instruments, a pre-analytical instrument will need to know whether the analytical instrument is ready to receive the pre-processed specimen from the pre-analytical instrument for analysis. Once a result is generated, the IT system, such as, for example, the laboratory information system (LIS), can be informed. Laboratory systems may also need information about the laboratory users so that the laboratory systems can notify and provide the laboratory information that best fits the role of each laboratory user.

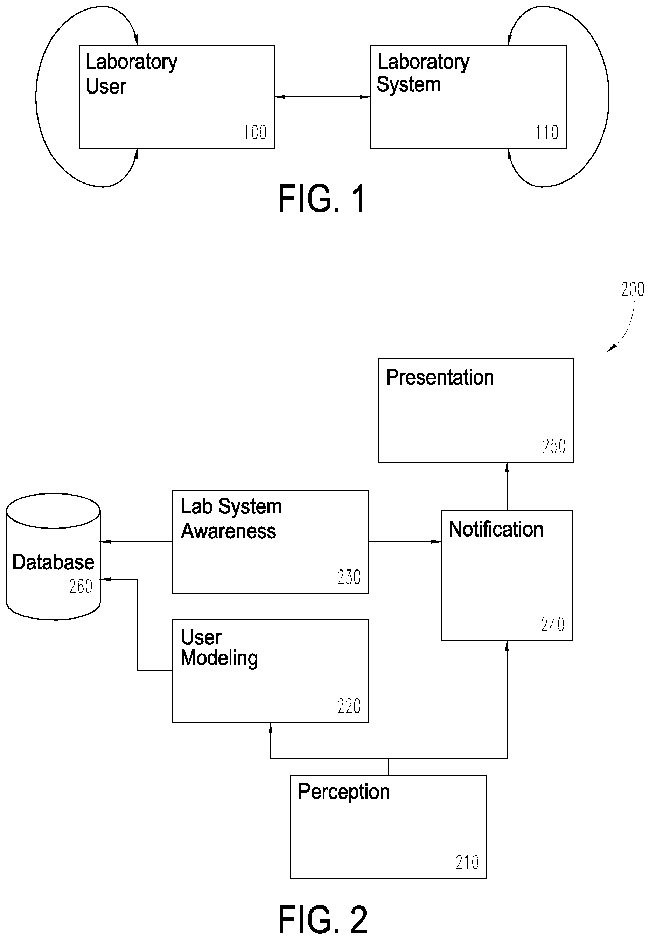

Referring initially to Fig. 1, Fig. 1 illustrates how information flows to address the needs of the different entities in the laboratory. As illustrated, a laboratory user 100 provides information to himself/herself and to other laboratory users as well as provides information to the laboratory system 110. And conversely, a laboratory system 110 provides information to itself and to other laboratory instruments within the laboratory system (i.e., pre-analyzers, analyzers, post-analyzers, and combinations thereof) as well as to laboratory user(s) 100. An ideal smart laboratory system 110 can facilitate the information interactions as shown in Fig. 1. The laboratory automation systems 110 of today are born to support the interactions between the different laboratory components.

To be concise, laboratory users 100 can work in a coordinated manner, either with laboratory systems 110 as well as with other laboratory user(s) 100. This indicates that there needs to be a certain level of "awareness" between the laboratory users and the laboratory system. Here, awareness refers to being aware of what other laboratory users are doing and what the laboratory instruments systems are doing. It is important to support the laboratory user's awareness by pushing the right information to the right laboratory user to do the right task at the right time and at the right location in the laboratory.

This can be translated to four factors of context-awareness, namely, subject, location, activity, and time (or SLAT contexts). By exploitation of the SLAT contexts, better work coordination in the laboratory can be achieved.

It is also important to differentiate between public and private information spaces of the laboratory user for that interaction.

A private space is an interactive space in which each individual laboratory user interacts privately. In this private space, information is only consumed privately by the laboratory user and that laboratory user needs to be a registered laboratory user of the laboratory system for security concerns as well as to maintain the integrity of the information. The private space provides information and notifications that are only interesting for a particular laboratory user, or particular group of laboratory users. It is also possible to provide confidential information in the private space. However, the laboratory user has the option to actively choose to "upload" information from the laboratory user's private space to the public space if such a need arises. If the laboratory user opts to upload information from the private space of the laboratory user to the public space, the laboratory user registration will accompany the upload for error tracking purposes, i.e., the wrong private information is uploaded or uploaded to the wrong location in the public space.

In contrast, the public space can be an interactive space in which each individual laboratory user, or consumer, can interact publicly. The information in the public space can also be consumed publicly. In other words, all laboratory users are able to perceive, i.e., see and/or hear the information in the public space. Public information can also be transferred and/or saved into a private space of a laboratory user. In the public space, the information is primarily visible to all laboratory users to increase their awareness of certain laboratory information. The laboratory user can have the option to "download" information from the public space to a private space if such a need arises. Again, only registered laboratory user will have the ability to download information from the public space to the private space for security reasons as well as to preserve the information integrity. Additionally, the laboratory user can augment the information in the private space of the laboratory user. The most relevant laboratory information can be automatically be pushed into the public space of the laboratory user based on the proximity of that laboratory user.

One of the main advantages of the present disclosure is that efficiency of work in the laboratory can be increased with these intuitive interactions. In other words, the smart laboratory system can be aware of 1) who is interested in what, 2) who has which skill sets, 3) who is working on which tasks at which location, and 4) who needs what information at what specific time.

As a result, if there is an event for which a laboratory user needs to take care of, the smart laboratory system is capable of judging who is the right person to notify and how (e.g., via the private or public notifications). If the laboratory user is performing a task, the smart laboratory system also has the capability of judging what information the laboratory user may need at each step of the task.

This system and method provides a solution that can help achieve maximum efficiency in the laboratory without relying only on specific laboratory roles in certain areas and can help support the laboratory system by enhancing tasks at a maximum performance for multidisciplinary teams.

Turning now to Fig. 2, Fig. 2 illustrates the architecture 200 of the smart laboratory system. The architecture comprises of five main components.

The first component is the perception component 210. The perception component 210 provides information regarding situational/environment data occurring outside the laboratory system. In this component 210, the smart laboratory employs various means to extract in-situ SLAT (Subject, Location, Activity, and Time) context data such as, for example, identification, location, motion, duration, and interaction. In one embodiment, the laboratory users can be identified by log-on data on laboratory devices. In another embodiment, the laboratory users can be identified by wearable devices of the laboratory users. Indoor positioning systems can be used to extract the absolute location of a laboratory user. Based on the layout of the laboratory, the absolute locations of laboratory users can be translated to locations relative to predefined positions such as, for example, laboratory instrument locations, desks, or any other relevant objects in the laboratory. Motion sensors within the laboratory can also be used in order to detect motion, i.e., the direction of moving of a laboratory user. Such information can be computed with tracked real-time location. The location data can also be used to compute time spent at certain locations. The interaction data on a screen can be used to compute time spent on a particular screen. Additionally, laboratory user interactions with laboratory systems may also provide context regarding what activities the laboratory user is currently engaging in.

The second component is the user modeling component 220. In this component 220, each laboratory user can be modeled. An a priori model is initially built based on known data of each laboratory user such as, for example, demographics, preferences, and role. This user model can be configured continuously with the in-situ context information received from the perception component 210. This way, each laboratory user can be modeled using the work habits and activities of the laboratory user. The user model can be updated as the laboratory system learns about each laboratory user.

The third component is the laboratory system awareness component 230. The laboratory system awareness component 230 provides information regarding data occurring within the laboratory system itself. In this component 230, the laboratory system operates and is self-aware of what it is currently being performed (i.e., status), how well the laboratory system is performing (i.e., performance), if laboratory user intervention is needed in the laboratory (i.e., alerts and alarms), and what documentation the laboratory user may need during interaction with the laboratory (i.e., help). All this information is fed or communicated to the fourth component, the notification logic component 340.

The notification logic component 240 includes an ever-changing in-situ contextual state, which keeps all laboratory users and laboratory systems, and an algorithm, which determines what, where, and how to show information to what laboratory user at a given time. The notification logic component 240 receives input from the perception component 210 and the laboratory awareness component 230 and provides instructions to the fifth component, the presentation component 250, on how to present the information. The algorithm can be supported with machine learning in order to enrich itself through use and can be improved as the algorithm gains experience through that usage. The use of the algorithm can create secure databases in order to generate statistics and/or projections and provide improvements in services between the clients and suppliers.

The fifth component is the presentation component 250. In this component 250, all possible ways of presentations are defined for both the public and private space notifications. The public space notifications can include, for example, monitoring displays positioned throughout the laboratory, voice assistants positioned throughout the laboratory, laboratory device displays, and alarms. The private space notifications can include, for example, smart phones, tablets, laptops and desktops, wearables such as smart watches or Google glasses, and virtual space enabled via virtual or augmented reality. The laboratory user's private notification typically provides confidential information for that particular laboratory user. Laboratory users can toggle between the private and public presentation of notifications and data as needed. For example, the laboratory user can upload the data that is currently private notifications of the laboratory user to be public notifications of the laboratory user based on a laboratory user's request. Conversely, the laboratory user can also download the data that is currently public notifications of the laboratory user to be private notifications of the laboratory user. Laboratory users can exchange information between private and public spaces according to different data transfer mechanisms. For example, the data transfer could be through image processing and/or proximity connectivity.

In addition, the architecture 200 of the smart laboratory system can also comprise a database 260 for storing the in-situ context data from the perception component 210 and the laboratory device status data from the laboratory system awareness component 230. Additionally, the development and efficiency in which the task was performed by the laboratory user can be measured and stored on the database 260 by the smart laboratory system.

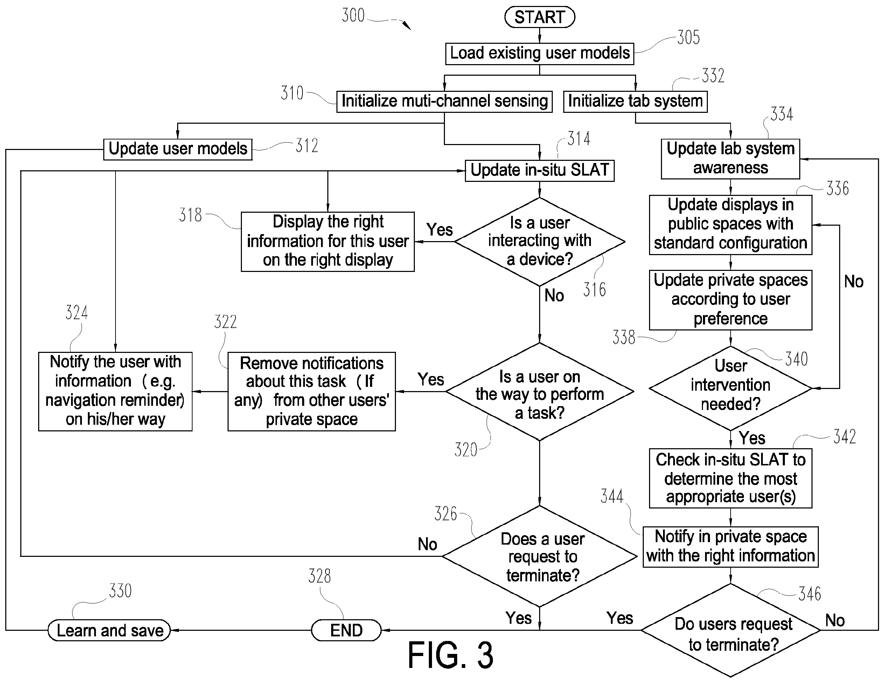

Fig. 3 illustrates a flow diagram of the method for presenting laboratory data to a laboratory user by troubleshooting using proximity interaction.

The method for presenting laboratory data to a laboratory user starts with loading a first initial user model in step 300. The first initial user model can be based on, for example, the laboratory user's individual demographics, preferences, and laboratory role.

In the next step 310, multi-channel sensors throughout a laboratory are initialized to collect in-situ data as the data occurs within the laboratory. The in-situ context data can be gathered from the multi-channel sensors, wherein the multi-channel sensors can be, for example, wearables, indoor positioning devices, motion sensors, laboratory user location data, laboratory user interactions, and combinations thereof. The in-situ data can comprise data concerning the laboratory user, the laboratory user's location, the laboratory user's activity, and the time.

In a next step 312, the user models are updated by using each individual laboratory user's work habits and usual laboratory activities as detected by the multi-channel sensors and stored in a database. Additionally, in step 314, the in-situ/SLAT data is updated and stored at the same time.

Next, in step 316, it is determined whether the laboratory user is interacting with any of the laboratory devices in the laboratory. If it is determined that the laboratory user is interacting with a laboratory device, the laboratory device information is provided to the laboratory user via the laboratory user's private and public notifications based on the laboratory user's user model. The information provided by the public and private notifications can be predefined by default. The public data can be thought of as the data that all laboratory users of the laboratory system may need to see such as, for example, operating instruction and helpful hints. This public data can be presented to the laboratory user, for example, on monitoring displays positioned throughout the laboratory, voice assistant devices, laboratory device displays, alarms, or any combination thereof. The private data can be presented to the laboratory user, for example, on the laboratory user's smart phones, tablets, laptops, desktops, wearable smart devices, virtual space, or any combination thereof. The laboratory user can also upload the data currently provided as a private notification to the laboratory user to be provided as a public notification of the laboratory user based on a laboratory user's request. In other words, if a laboratory user is detected to be interacting with laboratory devices, the right information will be pushed to the right method of notification to the laboratory user according to the user model of that particular laboratory user.

In addition, the development and efficiency in which the task was performed by the laboratory user is measured by the smart laboratory system under continuous improvement criteria in order to guarantee a constant optimal and effective performance. The development and efficiency in which the task was performed by the laboratory user can also be saved by the labortory system in, for example, a database.

In step 320, it can be determined whether the laboratory user is in transit to perform a task in a laboratory. If the laboratory user is detected to be on the way to perform a task, then information is pushed to the laboratory user's notification based on the SLAT (Subject, Location, Activity, and Time) of the laboratory user. Further, while the laboratory user is in transit to perform the task, the messages regarding that the task needs to be performed will be removed from the laboratory user's private notifications, in step 322, and the laboratory user will receive information on the task based on the updated in-situ data in step 324. The information can include navigational help, i.e., directions to location of the task, as well as operational instructions on how to perform the task, for example.

If the laboratory user is not interacting with any laboratory device or is not in transit to a laboratory task, the laboratory user, in 326, may wish to terminate the method. If the laboratory user does not wish to terminate the method, steps 314 through 326 are repeated until the laboratory user requests termination.

If the laboratory user does request termination in step 326, the in-situ data is saved in step 330 and the user specific model will be updated in step 312.

Concurrently to step 310, laboratory systems are initialized, in step 332, after the first initial user models are loaded.

After the laboratory systems are initialized, a laboratory device awareness component is updated, in step 334, with the current status of the laboratory, how well the laboratory is currently performing, if laboratory user intervention is needed and what alarms are need to alert the laboratory user of the needed intervention, and what documentation, i.e., help, may the laboratory user may need during interaction i.e., task performance, with a laboratory device in the laboratory.

After the laboratory device awareness component is updated, the public notifications to the laboratory user are updated, in step 336, with standard predefined configurations and the private notifications to the laboratory user are also updated, in step 338, according to laboratory user preferences.

In step 340, it is determined whether laboratory user intervention is needed. Laboratory user invention may be required based on several factors such as, for example, laboratory performance, laboratory instrument status, if trouble shooting a problem within a laboratory is required such as, for example, in the case of laboratory instrument failure or a laboratory user requires assistance, or any combination thereof. If no intervention is currently required by the laboratory user in the laboratory, steps 336 through 340 are repeated

If the laboratory system requires laboratory user intervention, the laboratory system can analyze and determine the right laboratory user(s) to notify, in step 342 by checking the current in-situ data to determine an appropriate laboratory user(s) to provide intervention and updating the private notifications to that laboratory user to provide appropriate laboratory user intervention information, in step 344.

In step 346, the laboratory system can be determined whether the laboratory user wishes to terminate the method. If the laboratory user does not want to terminate the method, steps 334 through 346 are repeated until it comes to the time that the laboratory user does request termination of the method.

If the laboratory user does request termination in step 346, the method is terminated in step 328, the in-situ data is saved in step 330 and the user specific model will be updated in step 312.

### Use Cases

### Notifying users privately based on SLAT

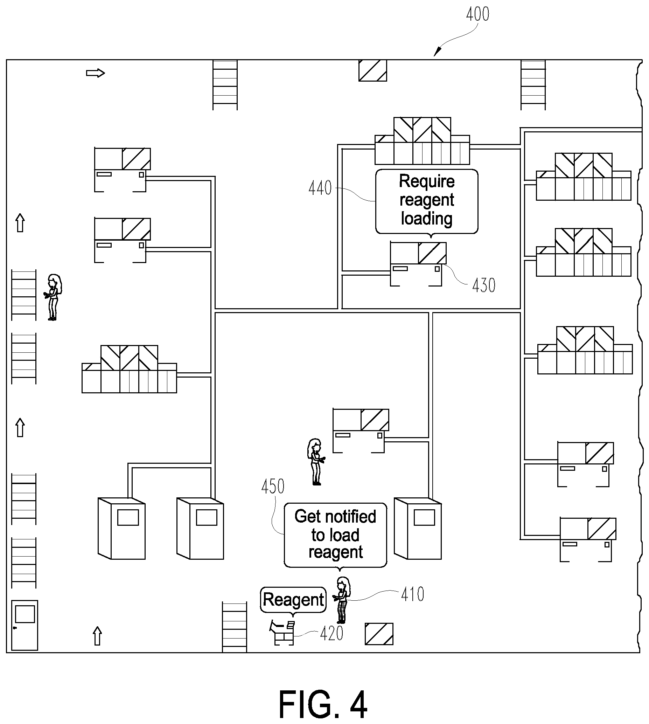

Fig. 4 presents the use case where a laboratory technician 410 has just finished one laboratory task and is walking in the laboratory 400, passing by the reagent storage area 420. At this time, one laboratory device 430 in the laboratory 400 requires reagents to be loaded. The public notifications can be provided, for example, on displays 440, including monitoring displays, i.e., displays spread throughout the laboratory, as well as the display of the laboratory device 430 needing reagents provide this notification publicly. Since the laboratory technician 410 is currently idle and is located closest amongst all the laboratory technicians in the laboratory 400 to the reagent storage 420, the laboratory technician 410 receives a private notification on his/her device 450. Then, this laboratory technician 410 can decide to take this task. If the laboratory technician 410 decides to take on this task, the laboratory technician 410 can retrieve the necessary full reagents from the reagent storage 420 and can move towards the laboratory device requiring new reagents 430. Since the smart laboratory system knows the laboratory technician 410 has accepted the task and is going to fulfil the task, every time the laboratory technician 410 passes a monitoring display in the laboratory 400, the monitoring display provides the direction in the laboratory 400 of the laboratory device needing reagents 430 to the laboratory technician 410. The direction provided to the laboratory technician 410 may be indicated visually such as by direction arrows displayed on the monitors of instruments or by flashing lights, audibly such as by the laboratory device requiring service 430 emitting a sound such as a beep, tactilely such as by sensors place throughout the laboratory 400, or by any other potential indicator that would allow the laboratory technician 410 to easily locate the laboratory device needing reagents 430 in the laboratory 400.

### Supporting novice users

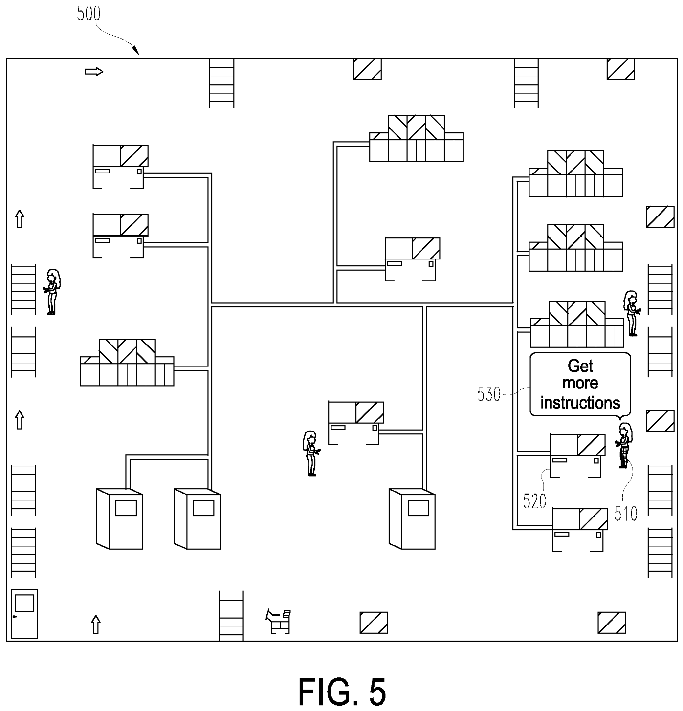

As shown in Fig. 5, a laboratory intern 510 has joined the laboratory 500 for a week. Although the laboratory intern 510 has been trained how to use the laboratory device 520, the laboratory intern 510 is far from proficient. The smart laboratory system recognizes that the laboratory intern 510 is a novice based on the user profile of the laboratory intern 510. When the laboratory intern 510 interacts with the laboratory device 520, especially while completing complex tasks, the laboratory intern 510 receives additional help or guidance 530 in order to complete the task. The user profile of the laboratory intern 510 can evolve automatically as the laboratory intern 510 becomes more proficient. As the system is continuously evaluating the performance and learning of the laboratory intern 510, at some point, the guidance for the novice users will no longer be shown.

### Supporting service representatives

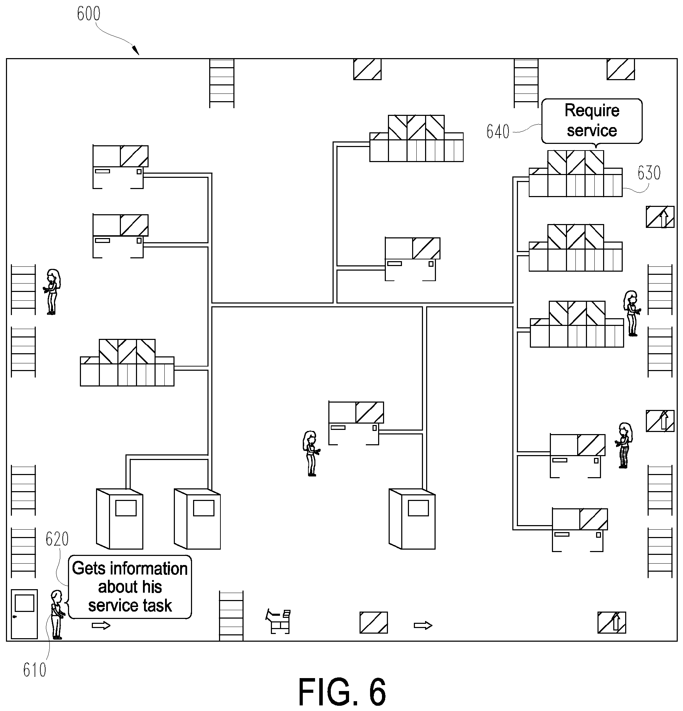

As shown in Fig. 6, after a service representative 610 enters a laboratory 600, the service representative 610 receives service tasks 620 from his private space device. The service tasks 620 will not be provided on the public monitoring devices because the service representative 610 is the only service user in the laboratory 600. Similar to the user case shown in Fig. 4, as the service representative 610 walks to the laboratory device requiring service 630, the public monitoring devices such as, for example, monitor screens, will indicate the direction in the laboratory 600 of the device requiring service 630. The direction may be indicated visually such as by direction arrows displayed on the monitors of instruments or by flashing lights, audibly such as by the laboratory device requiring service 630 emitting a sound such as a beep, tactilely such as by sensors place throughout the laboratory 600, or by any other potential indicator that would allow the service representative 610 to easily locate the laboratory device requiring service 630 in the laboratory 600. When the service representative 610 arrives at the laboratory device requiring service 630, the laboratory device display 640 will provide more details related to the service tasks, issues, possible guidance, and the like. For example, the details may be provided on a monitor screen of the laboratory device requiring service 630. The service representative 610 can then download critical technical details to his private space by tapping his device on the laboratory device 630 so that the service representative 610 can search for more information concerning that laboratory device 630 if needed.

### Supporting Laboratory Managers

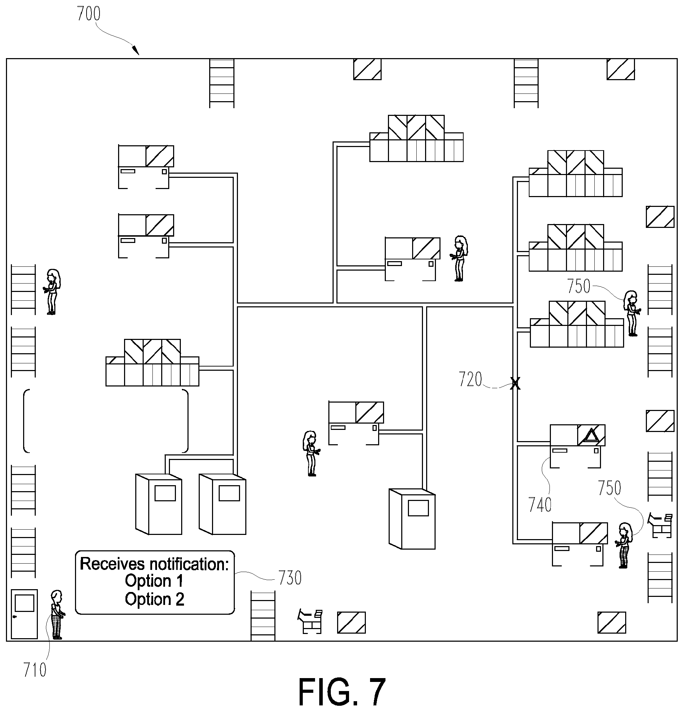

As shown in Fig. 7, a laboratory manager 710 makes critical decisions continuously based on the condition of the laboratory 700 under management of the laboratory manager 710 in order to ensure that the laboratory 700 delivers results efficiently. For example, the laboratory manager 710 may be notified privately by the smart laboratory system that the throughput of the laboratory 700 has dropped significantly. This may occur, for example, due to a transportation jam 720 occurring en route to an analyzer 740 so that laboratory samples are not arriving at the analyzer 740 in a timely manner.

The smart laboratory system can then provide to the laboratory manager 710 via private notification 730 of at least two options as backup solutions to increase throughput based on smart algorithms. The laboratory manager 710 can, then, decided between the provided options and assign a responsible person in the laboratory 700, for example, a laboratory technician 750, to rectify the problem. The smart laboratory system can then send a notification to that responsible person 750 so that that person 750 is aware of the decision by the laboratory manager 710. Such decisions by the laboratory manager 710 can be made remotely without having to approach the laboratory instrument 740 in the laboratory 700 or to even be in the laboratory area 700. These decisions can be based on previous choices made by the laboratory manager 710 as well as options that the smart laboratory system considers appropriate and that could improve overall laboratory performance.

Further disclosed is a computer program product comprising instructions which, when executed by a control unit of an analytical laboratory, can cause the analytical laboratory to perform the steps of any one of the methods disclosed herein. Thus, specifically, one, more than one or even all of the method steps as disclosed herein may be performed by using a computer or a computer network (such as a cloud computing service) or any suitable data processing equipment. As used herein, a computer program product can refer to the program as a tradable product. The product may generally exist in any format, such as in a downloadable file, on a computer-readable data carrier on premise or located at a remote location (cloud). The computer program product may be stored on a non-transitory computer-readable data carrier; a server computer as well as on transitory computer-readable data carrier such as a data carrier signal. Specifically, the computer program product may be distributed over a data network. Furthermore, not only the computer program product, but also the execution hardware may be located on-premise or remotely, such as in a cloud environment.

Further disclosed and proposed is a non-transitory computer-readable storage medium comprising instructions which, when executed by a control unit of an analytical laboratory, can cause the analytical laboratory to perform the steps of any one of the methods disclosed herein.

Further disclosed and proposed is a modulated data signal comprising instructions which, when executed by a control unit of an analytical laboratory, can cause the analytical laboratory to perform the steps of any one of the methods disclosed herein.

It is noted that terms like "preferably," "commonly," and "typically" are not utilized herein to limit the scope of the claimed embodiments or to imply that certain features are critical, essential, or even important to the structure or function of the claimed embodiments. Rather, these terms are merely intended to highlight alternative or additional features that may or may not be utilized in a particular embodiment of the present disclosure.

For the purposes of describing and defining the present disclosure, it is noted that the term "substantially" is utilized herein to represent the inherent degree of uncertainty that may be attributed to any quantitative comparison, value, measurement, or other representation. The term "substantially" is also utilized herein to represent the degree by which a quantitative representation may vary from a stated reference without resulting in a change in the basic function of the subject matter at issue.

Having described the present disclosure in detail and by reference to specific embodiments thereof, it will be apparent that modifications and variations are possible without departing from the scope of the disclosure defined by the appended claims. More specifically, although some aspects of the present disclosure are identified herein as preferred or particularly advantageous, it is contemplated that the present invention is not necessarily limited to these preferred aspects of the disclosure.

## Claims

1. A system (200) for presenting laboratory data to one or more laboratory users (100), the system (200) comprising:
a perception component (210) configured to continuously gather in-situ context data regarding a laboratory and the laboratory user(s) (100);
a user modeling component (220) configured to communicatively receive the in-situ context data from the perception component (210) to create a user specific model for each laboratory user (100) in the laboratory;
a laboratory device awareness component (230) configured to monitor the status, performance, alarms, and/or maintenance of the laboratory devices within the laboratory;
a notification component (240) configured to communicatively receive the in-situ context data from the perception component (210) and the laboratory device status data from the laboratory device awareness component (230), the laboratory device status data including the status, performance, alarms and/or maintenance of the laboratory devices, and to process and determine which of and how these data from the in-situ context data and the laboratory device status data are to be presented to what laboratory user (100) based on the in-situ context data and the user specific model(s); and
a presentation component (250) communicatively connected to the notification component (240) and configured to present the processed data from the notification component (240) to a laboratory user (100), wherein the presented data comprises both public and private presentations of the data to the laboratory user (100);
wherein the presentation component (250) is configured to:
privately notify a laboratory manager (710) of a reduction in the throughput of the laboratory (700); and
provide a private notification (730) to the laboratory manager (710) of at least two options as backup solutions to increase the laboratory throughput based on algorithms, such that the laboratory manager (710) can decide between said at least two options and assign a responsible person (750) to rectify the reduction in throughput; and
wherein the system (200) is configured to send a notification to the responsible person (750) to make said responsible person (750) aware of the option decided on by the laboratory manager (710).

2. The system (200) according to claim 1, wherein the in-situ context data is gathered from multi-channel sensors such as wearables, indoor positioning devices, motion sensors, laboratory user location data, laboratory user interactions with the laboratory, and combinations thereof.

3. The system (200) according to claims 1 and 2, wherein the laboratory user (100) toggles between the private and public notifications of data.

4. The system (200) according to any one of the preceding claims, wherein the private and public data notifications are predefined.

5. The system (200) according to any one of the preceding claims, wherein the private data is presented to the laboratory user (100) on smart phones, tablets, laptops, desktops, wearable smart devices, virtual space, or any combination thereof.

6. The system (200) according to any one of the preceding claims, wherein the public data is presented to the laboratory user (100) on monitoring displays positioned throughout the laboratory, voice assistant devices positioned throughout the laboratory, laboratory device displays, alarms, or any combination thereof.

7. The system (200) according to any one of the preceding claims, wherein the public data are data that all laboratory users (100) of the system may see.

8. The system (200) according to any one of the preceding claims, further comprises, a database (260) for storing the in-situ context data received from the perception component (210) and the laboratory device status data from the laboratory system awareness component (230).

9. A computer-implemented method (300) for presenting laboratory data to a laboratory user (100), the method (300) comprising:
a) loading a first initial user model;
b) initializing multi-channel sensors positioned throughout a laboratory to collect in-situ context data as the data occurs;
c) updating user models by using each individual laboratory user's in-situ context data detected by the multi-channel sensors;
d) updating the in-situ context data;
e) determining if the laboratory user (100) is interacting with a laboratory device with the laboratory;
f) if the laboratory user (100) is interacting with a laboratory device located within the laboratory, providing device information to the laboratory user (100) with private and public notifications based on the laboratory user's user model, including:
providing a private notification (730) to a laboratory manager (710) notifying a reduction in the throughput of the laboratory (700);
providing a private notification (730) to the laboratory manager (710) of at least two options as backup solutions to increase the laboratory throughput based on algorithms, such that the laboratory manager (710) can decide between said at least two options and assign a responsible person (750) to rectify the reduction in throughput; and
sending a notification to the responsible person (750) to make said responsible person (750) aware of the option decided on by the laboratory manager (710);
g) determining if the laboratory user (100) is in transit to perform a task in a laboratory;
h) if the laboratory user (100) is in transit, removing notifications regarding the task to be performed from the laboratory user's private notifications and apprising the laboratory user with information on the task based on the updated in-situ context data;
i) terminating the method (300) if requested by the laboratory user (100) or otherwise repeat steps c) through h) until the laboratory user (100) requests termination; and
j) saving the in-situ context data and updating the user specific model.

10. The computer-implemented method (300) according to claim 9, further comprising, measuring and storing efficiency in a database (260) as to how well the laboratory user (100) completes the task.

11. The computer-implemented method (300) according to any one of claims 9 - 10, further comprising, uploading data currently as a private notification to the laboratory user (100) in order to also be provided as a public notification to the laboratory user (100) based on the laboratory user's request.

12. The computer-implemented method (300) according to any one of claims 9 - 11, wherein the first initial user model is based on the laboratory user's individual demographics, preferences, and laboratory role.

13. The computer-implemented method (300 according to any one of claims 9 - 12, wherein the in-situ data comprises data of the laboratory user 100, the laboratory user's location, the laboratory user's activity, and the time.

14. The computer-implemented method (300) according to any one of claims 9 - 13, wherein the laboratory user's private notifications provide confidential information for the laboratory user (300).

15. The computer-implemented method (300) according to any one of claims 9 - 14, further comprising,
k) initializing laboratory systems after loading the first initial user models;
I) updating a laboratory device awareness model;
m) updating the laboratory user's public notifications with standard predefined configurations;
n) updating the laboratory user's private notifications according to the laboratory user preferences;
o) determining if intervention is needed by the laboratory user (100);
p) if no intervention is required from the laboratory user (100), repeating steps m) through o);
q) if intervention is required from the laboratory user (100), checking the current in-situ context data to determine an appropriate laboratory user (100) to provide intervention and updating the private notifications to that laboratory user (100) to provide appropriate user information;
r) determining if the laboratory user (100) wishes to terminate the method (300); and
s) if the laboratory user (100) does not want to terminate the method (300), repeating steps l) through r) until the laboratory user (100) wants to terminate the method (300).

## Patentansprüche

1. System (200) zur Darstellung von Labordaten für einen oder mehrere Laborbenutzer (100), wobei das System (200) umfasst:
eine Wahrnehmungskomponente (210), die konfiguriert ist, um kontinuierlich In-situ-Kontextdaten bezüglich eines Labors und des/der Laborbenutzer(s) (100) zu erfassen;
eine Benutzermodellierungskomponente (220), die konfiguriert ist, um die In-situ-Kontextdaten von der Wahrnehmungskomponente (210) kommunikativ zu empfangen, um für jeden Laborbenutzer (100) im Labor ein benutzerspezifisches Modell zu erstellen;
eine Komponente zum Überblicken von Laborvorrichtungen (230), die konfiguriert ist, um den Status, die Leistung, die Alarme und/oder die Wartung der Laborvorrichtungen innerhalb des Labors zu überwachen;
eine Benachrichtigungskomponente (240), die konfiguriert ist, um die In-situ-Kontextdaten von der Wahrnehmungskomponente (210) und die Statusdaten der Laborvorrichtungen von der Komponente zum Überblicken von Laborvorrichtungen (230) kommunikativ zu empfangen, wobei die Statusdaten der Laborvorrichtungen den Status, die Leistung, die Alarme und/oder die Wartung der Laborvorrichtungen umfassen, und um zu verarbeiten und zu bestimmen, welche dieser Daten aus den In-situ-Kontextdaten und den Statusdaten der Laborvorrichtungen und in welcher Form welchem Laborbenutzer (100) auf der Grundlage der In-situ-Kontextdaten und des/der benutzerspezifischen Modells/Modelle darzustellen sind; und
eine Darstellungskomponente (250), die kommunikativ mit der Benachrichtigungskomponente (240) verbunden und konfiguriert ist, um die verarbeiteten Daten von der Benachrichtigungskomponente (240) einem Laborbenutzer (100) darzustellen, wobei die dargestellten Daten sowohl öffentliche als auch vertrauliche Darstellungen der Daten für den Laborbenutzer (100) umfassen;
wobei die Darstellungskomponente (250) konfiguriert ist, um:
einen Laborleiter (710) vertraulich über eine Verringerung des Durchsatzes des Labors (700) zu benachrichtigen; und
dem Laborleiter (710) eine vertrauliche Benachrichtigung (730) mit mindestens zwei Optionen als Ausweichlösungen zur Steigerung des Durchsatzes des Labors auf der Grundlage von Algorithmen bereitzustellen, sodass der Laborleiter (710) zwischen den mindestens zwei Optionen entscheiden und eine verantwortliche Person (750) benennen kann, um die Verringerung des Durchsatzes zu beheben; und
wobei das System (200) konfiguriert ist, um eine Benachrichtigung an die verantwortliche Person (750) zu senden, um die zuständige Person auf die vom Laborleiter (710) getroffene Entscheidung aufmerksam zu machen.

2. System (200) nach Anspruch 1, wobei die In-situ-Kontextdaten von Mehrkanalsensoren wie Wearables, Indoor-Ortungsvorrichtungen, Bewegungssensoren, Standortdaten von Laborbenutzern, Interaktionen von Laborbenutzern mit dem Labor und Kombinationen davon erfasst werden.

3. System (200) nach den Ansprüchen 1 und 2, wobei der Laborbenutzer (100) zwischen vertraulichen und öffentlichen Benachrichtigungen über Daten umschaltet.

4. System (200) nach einem der vorstehenden Ansprüche, wobei die vertraulichen und öffentlichen Datenbenachrichtigungen vordefiniert sind.

5. System (200) nach einem der vorstehenden Ansprüche, wobei die vertraulichen Daten dem Laborbenutzer (100) auf Smartphones, Tablets, Laptops, Desktop-Computern, tragbaren Smart-Vorrichtungen, in einem virtuellen Raum oder einer beliebigen Kombination davon dargestellt werden.

6. System (200) nach einem der vorstehenden Ansprüche, wobei die öffentlichen Daten dem Laborbenutzer (100) auf im gesamten Labor positionierten Überwachungsanzeigen, im gesamten Labor positionierten Sprachassistenzvorrichtungen, Anzeigen von Laborvorrichtungen, Alarmen oder einer beliebigen Kombination davon angezeigt werden.

7. System (200) nach einem der vorstehenden Ansprüche, wobei die öffentlichen Daten Daten sind, die alle Laborbenutzer (100) des Systems sehen können.

8. System (200) nach einem der vorstehenden Ansprüche umfasst ferner eine Datenbank (260) zum Speichern der von der Wahrnehmungskomponente (210) empfangenen In-situ-Kontextdaten und der von der Komponente zum Überblicken von Laborvorrichtungen (230) empfangenen Statusdaten der Laborvorrichtungen.

9. Computerimplementiertes Verfahren (300) zum Darstellen von Labordaten für einen Laborbenutzer (100), wobei das Verfahren (300) umfasst:
a) Laden eines ersten anfänglichen Benutzermodells;
b) Initialisieren von Mehrkanalsensoren, die im gesamten Labor positioniert sind, um In-situ-Kontextdaten zu sammeln, sobald diese anfallen;
c) Aktualisieren von Benutzermodellen unter Verwendung der von den Mehrkanalsensoren detektierten In-situ-Kontextdaten jedes einzelnen Laborbenutzers;
d) Aktualisieren der In-situ-Kontextdaten;
e) Bestimmen, ob der Laborbenutzer (100) mit einer Laborvorrichtung im Labor interagiert;
f) wenn der Laborbenutzer (100) mit einer im Labor befindlichen Laborvorrichtung interagiert, Bereitstellen von Vorrichtungsinformationen für den Laborbenutzer (100) in Form von vertraulichen und öffentlichen Benachrichtigungen auf der Grundlage des Benutzermodells des Laborbenutzers, einschließlich:
Bereitstellen einer vertraulichen Benachrichtigung (730) für einen Laborleiter (710), die ihn über eine Verringerung des Durchsatzes des Labors (700) benachrichtigt;
Bereitstellen einer vertraulichen Benachrichtigung (730) für einen Laborleiter (710) mit mindestens zwei Optionen als Ausweichlösungen zur Steigerung des Durchsatzes des Labors auf der Grundlage von Algorithmen, sodass der Laborleiter (710) zwischen den mindestens zwei Optionen entscheiden und eine verantwortliche Person (750) benennen kann, um die Verringerung des Durchsatzes zu beheben; und
Senden einer Benachrichtigung an die verantwortliche Person (750), um die verantwortliche Person (750) auf die vom Laborleiter (710) getroffene Entscheidung zu aufmerksam zu machen;
g) Bestimmen, ob der Laborbenutzer (100) unterwegs ist, um eine Aufgabe in einem Labor auszuführen;
h) wenn der Laborbenutzer (100) unterwegs ist, Entfernen von Benachrichtigungen bezüglich der auszuführenden Aufgabe aus den vertraulichen Benachrichtigungen des Laborbenutzers und Unterrichten des Laborbenutzers über Informationen zur Aufgabe auf der Grundlage der aktualisierten In-situ-Kontextdaten;
i) Beenden des Verfahrens (300), wenn dies vom Laborbenutzer (100) angefordert wird, oder andernfalls Wiederholen der Schritte c) bis h), bis der Laborbenutzer (100) das Beenden anfordert; und
j) Speichern der In-situ-Kontextdaten und Aktualisieren des benutzerspezifischen Modells.

10. Computerimplementiertes Verfahren (300) nach Anspruch 9, das ferner das Messen und Speichern von Effizienz in einer Datenbank (260) hinsichtlich der Qualität der Aufgabenerledigung durch den Laborbenutzer (100) umfasst.

11. Computerimplementiertes Verfahren (300) nach einem der Ansprüche 9 bis 10, das ferner das Hochladen von Daten umfasst, die derzeit als vertrauliche Benachrichtigung an den Laborbenutzer (100) gesendet werden, damit sie auf Anforderung des Laborbenutzers auch als öffentliche Benachrichtigung dem Laborbenutzer (100) bereitgestellt werden.

12. Computerimplementiertes Verfahren (300) nach einem der Ansprüche 9 bis 11, wobei das erste anfängliche Benutzermodell auf den individuellen demografischen Daten, Präferenzen und der Laborrolle des Laborbenutzers basiert.

13. Computerimplementiertes Verfahren (300) nach einem der Ansprüche 9 bis 12, wobei die In-situ-Daten Daten des Laborbenutzers (100), den Standort des Laborbenutzers, die Aktivität des Laborbenutzers und die Zeit umfassen.

14. Computerimplementiertes Verfahren (300) nach einem der Ansprüche 9 bis 13, wobei die vertraulichen Benachrichtigungen des Laborbenutzers vertrauliche Informationen für den Laborbenutzer (300) bereitstellen.

15. Computerimplementiertes Verfahren (300) nach einem der Ansprüche 9 bis 14, das ferner umfasst:
k) Initialisieren von Laborsystemen nach dem Laden der ersten anfänglichen Benutzermodelle;
l) Aktualisieren eines Modells zum Überblicken von Laborvorrichtungen;
m) Aktualisieren der öffentlichen Benachrichtigungen des Laborbenutzers mit vordefinierten Standardkonfigurationen;
n) Aktualisieren der vertraulichen Benachrichtigungen des Laborbenutzers entsprechend den Präferenzen des Laborbenutzers;
o) Bestimmen, ob ein Eingreifen des Laborbenutzers (100) erforderlich ist;
p) wenn kein Eingreifen des Laborbenutzers (100) erforderlich ist, Wiederholen der Schritte m) bis o);
q) wenn ein Eingreifen des Laborbenutzers (100) erforderlich ist, Überprüfen der aktuellen In-situ-Kontextdaten, um einen geeigneten Laborbenutzer (100) zum Bereitstellen von Eingreifen zu bestimmen, und Aktualisieren der vertraulichen Benachrichtigungen an diesen Laborbenutzer (100), um angemessene Benutzerinformationen bereitzustellen;
r) Bestimmen, ob der Laborbenutzer (100) das Verfahren (300) beenden möchte; und
s) wenn der Laborbenutzer (100) das Verfahren (300) nicht beenden möchte, Wiederholen der Schritte l) bis r), bis der Laborbenutzer (100) das Verfahren (300) beenden möchte.

## Revendications

1. Système (200) de présentation de données de laboratoire à un ou plusieurs utilisateurs du laboratoire (100), le système (200) comprenant :
un composant de perception (210) configuré pour recueillir en continu des données contextuelles in situ concernant un laboratoire et le(s) utilisateur(s) du laboratoire (100) ;
un composant de modélisation de l'utilisateur (220) configuré pour recevoir en communication les données contextuelles in situ provenant du composant de perception (210) pour créer un modèle spécifique à l'utilisateur pour chaque utilisateur du laboratoire (100) dans le laboratoire ;
un composant de reconnaissance de dispositif de laboratoire (230) configuré pour surveiller l'état, la performance, les alarmes et/ou la maintenance des dispositifs de laboratoire au sein du laboratoire ;
un composant de notification (240) configuré pour recevoir en communication les données contextuelles in situ provenant du composant de perception (210) et les données d'état du dispositif de laboratoire provenant du composant de reconnaissance de dispositif de laboratoire (230), les données d'état du dispositif de laboratoire comprenant l'état, les performances, les alarmes et/ou la maintenance des dispositifs de laboratoire, et pour traiter et déterminer lesquelles de, et comment, ces données parmi les données contextuelles in situ et les données d'état du dispositif de laboratoire doivent être présentées à quel utilisateur du laboratoire (100) sur la base des données contextuelles in situ et du/des modèle(s) spécifique(s) à l'utilisateur ; et
un composant de présentation (250) connecté en communication avec le composant de notification (240) et configuré pour présenter les données traitées provenant du composant de notification (240) à un utilisateur du laboratoire (100), dans lequel les données présentées comprennent à la fois des présentations publiques et privées des données à l'utilisateur du laboratoire (100) ;
dans lequel le composant de présentation (250) est configuré pour :
notifier de manière privée un chef de laboratoire (710) d'une réduction du débit du laboratoire (700) ; et
fournir une notification privée (730) au chef de laboratoire (710) d'au moins deux options en tant que solutions de secours pour augmenter le débit du laboratoire sur la base d'algorithmes, de telle sorte que le chef de laboratoire (710) peut choisir entre lesdites au moins deux options et désigner une personne responsable (750) pour remédier à la réduction du débit ; et
dans lequel le système (200) est configuré pour envoyer une notification à la personne responsable (750) pour informer ladite personne responsable (750) de l'option choisie par le chef de laboratoire (710).

2. Système (200) selon la revendication 1, dans lequel les données contextuelles in situ sont recueillies des capteurs multicanaux tels que des dispositifs portables, des dispositifs de positionnement en intérieur, des capteurs de mouvement, des données de localisation d'utilisateur du laboratoire, des interactions d'utilisateurs du laboratoire avec le laboratoire, et des combinaisons de ceux-ci.

3. Système (200) selon les revendications 1 et 2, dans lequel l'utilisateur du laboratoire (100) alterne entre les notifications de données privées et publiques.

4. Système (200) selon l'une quelconque des revendications précédentes, dans lequel les notifications de données privées et publiques sont prédéfinies.

5. Système (200) selon l'une quelconque des revendications précédentes, dans lequel les données privées sont présentées à l'utilisateur du laboratoire (100) sur des téléphones intelligents, des tablettes électroniques, des ordinateurs portables, des ordinateurs de bureau, des dispositifs intelligents portables, un espace virtuel ou une combinaison quelconque de ceux-ci.

6. Système (200) selon l'une quelconque des revendications précédentes, dans lequel les données publiques sont présentées à l'utilisateur de laboratoire (100) sur des écrans de surveillance positionnés dans tout le laboratoire, des dispositifs d'assistance vocale positionnés dans tout le laboratoire, des écrans de dispositifs de laboratoire, des alarmes ou une combinaison quelconque de ceux-ci.

7. Système (200) selon l'une quelconque des revendications précédentes, dans lequel les données publiques sont des données que tous les utilisateurs du laboratoire (100) du système peuvent voir.

8. Système (200) selon l'une quelconque des revendications précédentes, comprenant en outre une base de données (260) pour stocker les données contextuelles in situ reçues du composant de perception (210) et les données d'état du dispositif de laboratoire provenant du composant de reconnaissance de système de laboratoire (230).

9. Procédé mis en œuvre par ordinateur (300) pour présenter les données de laboratoire à un utilisateur du laboratoire (100), le procédé (300) comprenant :
a) le chargement d'un premier modèle d'utilisateur initial ;
b) l'initialisation de capteurs multicanaux positionnés dans tout un laboratoire pour collecter les données contextuelles in situ au fur et à mesure que les données apparaissent ;
c) la mise à jour des modèles d'utilisateur en utilisant les données contextuelles in situ de chaque utilisateur du laboratoire individuel détectées par les capteurs multicanaux ;
d) la mise à jour des données contextuelles in situ ;
e) la détermination si l'utilisateur du laboratoire (100) interagit avec un dispositif de laboratoire au sein du laboratoire ;
f) si l'utilisateur du laboratoire (100) interagit avec un dispositif de laboratoire situé au sein du laboratoire, la fourniture d'informations de dispositif à l'utilisateur du laboratoire (100) avec des notifications privées et publiques sur la base du modèle d'utilisateur de l'utilisateur du laboratoire, comprenant :
la fourniture d'une notification privée (730) à un chef de laboratoire (710) notifiant une réduction du débit du laboratoire (700) ;
la fourniture d'une notification privée (730) au chef de laboratoire (710) d'au moins deux options en tant que solutions de secours pour augmenter le débit du laboratoire sur la base d'algorithmes, de telle sorte que le chef de laboratoire (710) peut choisir entre lesdites au moins deux options et désigner une personne responsable (750) pour remédier à la réduction du débit ; et
l'envoi d'une notification à la personne responsable (750) pour informer ladite personne responsable (750) de l'option choisie par le chef de laboratoire (710) ;
g) la détermination si l'utilisateur du laboratoire (100) est en transit pour effectuer une tâche dans un laboratoire ;
h) si l'utilisateur du laboratoire (100) est en transit, la suppression des notifications concernant la tâche à effectuer parmi les notifications privées de l'utilisateur du laboratoire et informer l'utilisateur du laboratoire de la tâche sur la base des données contextuelles in situ mises à jour ;
i) la terminaison du procédé (300) si l'utilisateur du laboratoire (100) le demande ou sinon la répétition des étapes c) à h) jusqu'à ce que l'utilisateur du laboratoire (100) demande la terminaison ; et
j) la sauvegarde des données contextuelles in situ et la mise à jour du modèle spécifique à l'utilisateur.

10. Procédé mis en œuvre par ordinateur (300) selon la revendication 9, comprenant en outre la mesure et le stockage dans une base de données (260) de l'efficacité avec laquelle l'utilisateur du laboratoire (100) accomplit la tâche.

11. Procédé mis en œuvre par ordinateur (300) selon l'une quelconque des revendications 9 à 10, comprenant en outre le téléchargement des données actuellement sous la forme d'une notification privée à l'utilisateur du laboratoire (100) afin qu'elles soient également fournies sous la forme d'une notification publique à l'utilisateur du laboratoire (100) sur la base de la demande de l'utilisateur du laboratoire.

12. Procédé mis en œuvre par ordinateur (300) selon l'une quelconque des revendications 9 à 11, dans lequel le premier modèle d'utilisateur initial est basé sur les données démographiques individuelles, les préférences et le rôle au laboratoire de l'utilisateur du laboratoire.

13. Procédé mis en œuvre par ordinateur (300) selon l'une quelconque des revendications 9 à 12, dans lequel les données in situ comprennent des données de l'utilisateur du laboratoire (100), l'emplacement de l'utilisateur du laboratoire, l'activité de l'utilisateur du laboratoire et le temps.

14. Procédé mis en œuvre par ordinateur (300) selon l'une quelconque des revendications 9 à 13, dans lequel les notifications privées de l'utilisateur du laboratoire fournissent des informations confidentielles à l'utilisateur du laboratoire (300).

15. Procédé mis en œuvre par ordinateur (300) selon l'une quelconque des revendications 9 à 14, comprenant en outre
k) l'initialisation des systèmes de laboratoire après le chargement des premiers modèles d'utilisateur initial ;
l) la mise à jour d'un modèle de reconnaissance de dispositif de laboratoire ;
m) la mise à jour des notifications publiques de l'utilisateur du laboratoire avec des configurations standard prédéfinies ;
n) la mise à jour des notifications privées de l'utilisateur du laboratoire en fonction des préférences de l'utilisateur du laboratoire ;
o) la détermination si une intervention est nécessaire par l'utilisateur du laboratoire (100) ;
p) si aucune intervention n'est nécessaire de la part de l'utilisateur du laboratoire (100), la répétition des étapes m) à o) ;
q) si une intervention est nécessaire de la part de l'utilisateur du laboratoire (100), la vérification des données contextuelles in situ actuelles afin de déterminer un utilisateur du laboratoire (100) approprié pour fournir l'intervention et la mise à jour des notifications privées de cet utilisateur du laboratoire (100) pour fournir les informations appropriées de l'utilisateur ;
r) la détermination si l'utilisateur du laboratoire (100) souhaite terminer le procédé (300) ; et
s) si l'utilisateur du laboratoire (100) ne souhaite pas terminer le procédé (300), la répétition des étapes l) à r) jusqu'à ce que l'utilisateur du laboratoire (100) souhaite terminer le procédé (300).
